# EUROPÄISCHE PATENTSCHRIFT

(11) **EP 0 575 361 B1**
(45) Veröffentlichungstag und Bekanntmachung des Hinweises auf die Patenterteilung: **26.06.1996**
(21) Anmeldenummer: 92905289.2
(22) Anmeldetag: 05.03.1992
(51) Int. Cl.: C07D 295/155, C07D 295/185, A61K 31/495

(54) **NEUE 1-ARYL-4-PIPERAZINYL-CYCLOHEXANCARBONSÄURENITRILE, IHRE HERSTELLUNG UND VERWENDUNG**
NEW 1-ARYL-4-PIPERAZINYL-CYCLOHEXANECARBOXYLIC ACID NITRILES, THEIR PRODUCTION AND THEIR USE
NOUVEAUX NITRILES D'ACIDE 1-ARYL-4-PIPERAZINYL-CYCLOHEXANOCARBOXYLIQUE, LEUR PREPARATION ET LEUR UTILISATION

(30) Priorität: 15.03.1991 DE 4108527
(43) Veröffentlichungstag der Anmeldung: 29.12.1993
(73) Patentinhaber: BASF Aktiengesellschaft, D-67063 Ludwigshafen (DE)
(72) Erfinder: TREIBER, Hans-Jörg, D-6835 Brühl (DE); HOFMANN, Hans, Peter, D-6703 Limburgerhof (DE); SZABO, Laszlo, D-6915 Dossenheim (DE)
(86) Internationale Anmeldenummer: EP9200488
(87) Internationale Veröffentlichungsnummer: WO9216516

(56) Entgegenhaltungen:
- EP-A- 0 034 415
- DE-A- 1 795 362
- GB-A- 1 452 413
- US-A- 2 722 529

## Beschreibung

Die vorliegende Erfindung betrifft 1-Aryl-4-piperazinyl-cyclohexancarbonsäurenitrile, Verfahren zu deren Herstellung und deren Verwendung als Arzneimittel.

Es sind bereits Derivate des 4-Amino-1-phenyl-cyclohexancarbonsäurenitrils bekannt. So sind in der ZA-PS 66/5399 Verbindungen mit Morpholin- und Mescalin-antagonisierenden Eigenschaften und in der DE-AS 17 93 383 Verbindungen mit spasmolytischen, blutdrucksenkenden und neuroleptischen Eigenschaften genannt. Ferner werden 4-Piperidinyl-1-phenylcyclohexancarbonsäurenitrile mit starker Antihistaminwirkung in der EP-OS 34 415 beschrieben.

Weiter ist bereits eine große Zahl substituierter Piperazine mit antiarrhythmischen oder durchblutungsfördernden Eigenschaften bekannt. Bei diesen handelt es sich vorwiegend um Benzhydryl- oder Diarylbutylpiperazine, von denen das Flunarizin die bekannteste Verbindung ist. Sie wird zur Therapie peripherer und cerebraler Durchblutungsstörungen verwendet (DE-OS 19 29 330, DE-OS 33 26 148, DE-OS 25 47 570, EP-OS 256 890, DE-OS 36 00 390, EP-OS 159 566, EP-OS 285 219).

Es wurde nun gefunden, daß 1-Aryl-4-piperazinyl-cyclohexancarbonsäurenitrile der Formel I 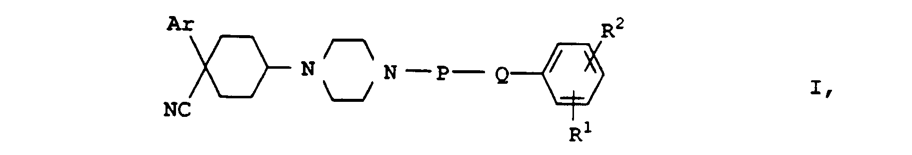 worin
- Ar: einen gegebenenfalls durch ein Chloratom, eine Methyl-, Methoxy- oder Trifluormethylgruppe, oder durch ein oder zwei Fluoratome substituierten Phenylrest, eine 2- oder 3-Thienylgruppe oder eine 2-, 3- oder 4-Pyridylgruppe,
- R¹: ein Wasserstoff-, Fluor- oder Chloratom oder eine Trifluormethyl-, Methoxy-, Methyl-, Nitro-, Cyano-, Acetyl-, Methoxycarbonyl- oder Ethoxycarbonylgruppe,
- R²: ein Wasserstoff- oder Chloratom oder eine Methylgruppe,
- P: ein geradkettiger oder verzweigter Kohlenwasserstoffrest mit 2 bis 8 C-Atomen, der durch eine Oxo-, Hydroxy, Methoxy- oder Acetoxygruppe substituiert sein kann, und
- Q: ein Sauerstoff- oder Schwefelatom, eine NH-Gruppe oder eine direkte Bindung
darstellen, sowie deren Salze mit physiologisch verträglichen Säuren eine hervorragende antihypoxische und antiischämische Wirkung besitzen.

Als physikalisch verträgliche Säuren kommen anorganische Säuren wie Salzsäure, Schwefelsäure, Phosphorsäure, Salpetersäure oder organische Säuren wie Weinsäure, Milchsäure, Apfelsäure, Zitronensäure, Maleinsäure, Fumarsäure, Oxalsäure, Essigsäure, Glukonsäure, Schleimsäure oder Methansulfonsäure in Betracht.

In der Formel I ist Ar vorzugsweise eine unsubstituierte oder eine durch ein Fluor- oder Chloratom substituierte Phenylgruppe, R¹ vorzugsweise ein Wasserstoff-, Fluor- oder Chloratom oder eine Methoxygruppe und P-Q insbesondere eine Oxy- oder Thioalkylengruppe mit 2 bis 3 Kohlenstoffatomen, sowie eine 2-Hydroxy-3-oxy-propylengruppe. Die Substituenten R¹ und R² können in den Positionen 2, 3 oder 4 am Phenylring stehen.

Die Verbindungen der Formel I besitzen, beispielsweise falls P eine Hydroxygruppe enthält, ein Chiralitätszentrum. Sie liegen daher in Form optischer Antipoden (Enantiomere) vor. Diese können nach gebräuchlichen Methoden durch Salzbildung mit chiralen Hilfskomponenten wie Weinsäure, Dibenzoylweinsäure oder Mandelsäure und fraktionierte Kristallisation und anschließende Freisetzung der Basen aus den Salzen oder auch synthetisch aus geeigneten chiralen Vorstufen erhalten werden.

Ferner können die Verbindungen wegen des Cyclohexanringes auch in Form von cis- und trans-Isomeren auftreten. Diese geometrischen Isomere können in an sich bekannter Weise durch Trennverfahren wie Kristallisation oder chromatographische Methoden auf der Endstufe oder einer Vorstufe oder auch durch geeignete Reakzionsführung erhalten werden.

Die Erfindung betrifft weiterhin Verfahren zur Herstellung der Verbindungen der Formel I, die dadurch gekennzeichnet sind, daß man entweder
a) eine Verbindung der Formel II 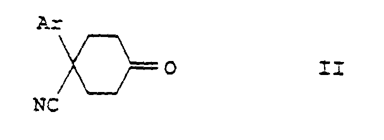 In welcher Ar die angegebene Bedeutung hat, mit einem Piperazinderivat III 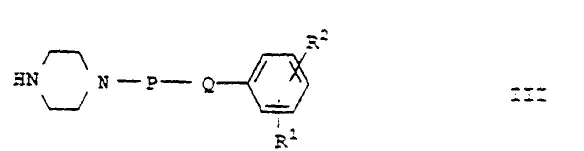 in welchem P, Q, R¹ und R² die angegebene Bedeutung haben, oder
b) eine Verbindung der Formel IV 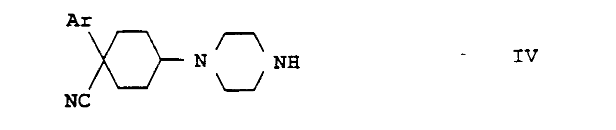 in welcher Ar wiederum die oben genannte Bedeutung hat, mit einer Verbindung der Formel V 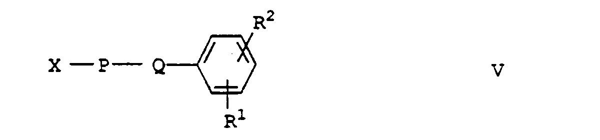 umsetzt, wobei P, R1 und R2 die obengenannte Bedeutung haben, und X einen reaktionsfähigen Säurerest darstellt, oder
c) - falls P durch eine Hydroxygruppe substituiert ist und Q keine NH-Gruppe darstellt - eine Verbindung der Formel IV mit einer Verbindung der Formel VI 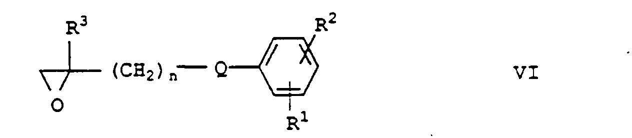 umsetzt, wobei Q, R¹ und R² die vorstehend genannte Bedeutung haben, R³ einen C₁₋₄-Alkylrest darstellt und n die Zahl 1 oder 2 bedeutet,
und die so erhaltenen Verbindungen gegebenenfalls anschließend mit physiologisch verträglichen Säuren in ihre Salze überführt.

Die an sich bekannte reduktive Aminierung von Verbindungen der Formel II mit Piperazinen der Formel III kann mit Reduktionsmitteln, wie katalytisch erregtem Wasserstoff, wobei vorzugsweise ein Edelmetallkazalysator wie z.B. Palladium auf Kohle verwendet wird, mit komplexen Metallhydriden wie z.B. Natrium-cyanborhydrid oder auch mit Ameisensäure in einem geeigneten Lösungsmittel bei Temperaturen von vorzugsweise 0 bis 100°C durchgeführt werden. Als Lösungsmittel eignen sich Acetonitril, Dimethylformamid, Tetrahydrofuran, Totuol oder niedere Alkohole wie Methanol oder Ethanol.

Je nach verwendeten Reduktionsmittel und den angewandten Bedingungen werden Gemische der cis- und trans-Isomeren oder auch das weitgehend reine, stabilere cis-Isomere erhalten.

Das verfaren b) wird vorzugsweise in polaren organischen Lösungsmitteln wie Alkoholen (z.B. Methanol, Ethanol, Isopropanol), einem niederen Keton (vorzugsweise Aceton, Methylethylketon oder Methylisobutylketon), in Dimethylformamid, Dimethylsulfoxid, Acetonitril oder gegebenenfalls auch in einem Kohlenwasserstoff wie Toluol vorteilhaft in Gegenwart einer Hilfsbase zum Abfangen der sich bildenden Säure (wie Natriumcarbonat, Kaliumcarbonat, Calciumcarbonat, Triethylamin oder Pyridin) bei erhöhter Temperatur, vorzugsweise zwischen 20 und 120°C durchgeführt. Als reaktionsfähige Säurereste X eignen sich Chlor-, Brom- oder Iodatome sowie Sulfonsäuregruppen, vorzugsweise Methansulfonyl-, Benzolsulfonuyl-, p-Toluolsulfonyl- oder Trifluormethansulfonylreste.

Die an sich bekannte Umsetzung von Epoxiden der Formel VI mit den Piperazinen der Formel IV wird zweckmäßigerweise in einem Lösungsmittel wie Acetonitril, Dimethylformamid, Tetrahydrofuran oder einem niederen Alkohol (vorzugsweise Methanol oder Ethanol) bei erhöhten Temperaturen (30 bis 120°C), vorzugsweise bei Siedetemperaturen des Lösungsmittels, durchgeführt. Werden optisch aktive Epoxide eingesetzt, erhält man die erfindungsgemaßen Verbindungen als reine Enantiomere.

Die Ausgangsverbindungen der Formeln II bis VI sind literaturbekannt oder lassen sich in an sich bekannter Weise herstellen.

Die erfindungsgemäßen Verbindungen eignen sich zur Behandlung von akuten und chronischen degenerativen Prozessen des zentralen Nervensystems und anderer Organe. Hierzu zählen insbesondere Erkrankungen, die auf eine akute oder chronische Störung der Blutversorgung oder des zellulären Stoffwechsels zurückzuführen sind, wie beispielsweise ischämische zerebrale Insulte, zerebrale Blutungen, vaskuläre Enzephalopatien und die daraus resultierenden dementiellen Erkrankungen, Hypoglykämien, Epilepsien, Migräne, traumatische Läsionen des Gehirns und des Rückenmarks, sowie die ischämiebedingte Degeneration des Herzens, der Nieren und anderer Organe, insbesondere auch im Zusammenhang mit Transplantationen und anderen operativen Eingriffen.

Die zerebroprotektive Wirkung der neuen Verbindungen wurde gegen die normobare hypoxische Hypoxie der Maus sowie gegen die globale zerebrale Ischämie der Maus erfaßt (Methoden in: H.P. Hofmann et al., Arzneim.-Forsch./Drug Res. 39, 304 - 308, 1989). Die neuen Substanzen erwiesen sich in beiden Modellen als ausgeprägt protektiv wirksam, was sich in der starken Verlängerung der Überlebenszeit Substanz-behandelter Tiere im Vergleich zu Placebo-behandelten Kontrolltieren zeigte.

Ebenso wurde die neuroprotektive Wirkung der neuen Substanzen am Modell der temporären Vorderhirnischämie an der Mongolischen Wüstenrennmaus nachgewiesen (Methode in: L. Szabo a. H.P. Hofmann, Arzneim.-Forsch./Drug Res. 39, 314 - 319, 1989). Bei Placebo-behandelten Kontrolltieren führt die kurzzeitige Unterbrechung der Blutversorgung zum Absterben Ischämie-empfindlicher Nervenzellen im Hippocanpus. Durch die Behandlung mit den neuen Verbindungen wird das Ausmaß der Neuronenschädigung deutlich vermindert, was einen relevanten Parameter für die antiischämische Wirksamkeit der neuen Substanzen darstellt.

Die erfindungsgemäßen Verbindungen können in üblicher Weise oral, rektal oder parenteral (subkutan, intravenös, intramuskulär, transdermal) verabfolgt werden.

Die Dosierung hängt vom Alter, Zustand und Gewicht des Patienten sowie von der Applikationsart ab. In der Regel beträgt die tägliche Wirkstoffdosis zwischen etwa 0,1 und 20 mg/kg Körpergewicht bei oraler Gabe und zwischen etwa 0,01 und 2 mg/kg Körpergewicht bei parenteraler Gabe. Im Normalfall werden mit täglichen Dosen von 10 bis 100 mg oral und 1 bis 10 mg parenteral zufriedenstellende Ergebnisse erzielt.

Die neuen Verbindungen können in den gebräuchlichen galenischen Applikationsformen fest oder flüssig angewendet werden, z.B. als Tabletten, Filmtabletten, Kapseln, Pulver, Granulate, Dragees, Suppositorien, Lösungen, Salben, Cremes, Sprays oder transdermale therapeutsche Systeme. Diese werden in üblicher Weise hergestellt. Die Wirkstoffe können dabei mit den üblichen galenischen Hilfsmitteln wie Tablettenbindern, Füllstoffen, Konservierungsstoffen, Tablettensprengmitteln, Fließreguliermitteln Antioxidantien und/oder Treibgasen verarbeitet werden (vgl. H. Sucker et al., Pharmazeutische Technologie, Thieme-Verlag, Stuttgart, 1978). Die so erhaltenen Applikationsformen enthalten den Wirkstoff normalerweise in einer Menge von 1 bis 75 Gew.-%.

### Beispiele

### A. Herstellung der Ausgangsverbindungen

a) 4- (4-Benzylpiperazinyl)1-phenyl-cyclohexancarbonsäurenitril
   35,0 g 4-Cyano-4-phenyl-cyclohexanon (0,176 Mol), (hergestellt nach J. Amer. Chem. Soc. 74, 773 (1972)) wurden mit 31 g 1-Benzylpiperazin (0,176 Mol) und 21 g Essigsäure in 200 ml Ethanol gelöst und bei Raumtemperatur eine Lösung von 12,5 g Natriumcyanoborhydrid (0,19 Mol) langsam zugetropft. Nach Rühren über Nacht wurde mit Wasser versetzt und mit Dichlormethan extrahiert. Nach dem Trocknen und Abdestillieren des Lösungsmittels hinterblieben 46,6 g (76 %) Öl.
   Durch Behandeln mit isopropanolischer Salzsäure wurde das Di-Hydrochlorid erhalten C₂₄N₂₉N₃ x 2 HCl, Fp. < 300°C. Das Produkt, das auf diese Weise erhalten wurde, bestand laut Dünnschichtchromatographie und NMR zu etwa gleichen Teilen aus dem cis- und dem transIsomeren.
b) Cis-4- (4-Benzylpiperazinyl)1-phenyl-cyclohexancarbonsäurenitril
   Die Isomerenmischung aus a) wurde in Essigsäureethylester gelöst und bis zu Beginn einer Trübung mit Hexan versetzt. Über Nacht kristallisierte die reine cis-Verbindung aus. Nach dem Absaugen wurden 15,5 g (33 %) erhalten. C₂₄H₂₉N₃, Fp. 120 - 123°C.
c) Cis-4-(4-Benzylpiperazinyl)1-phenyl-cyclohexancarbonsäurenitril
   5 g 4-Cyano-4-phenyl-cyclohexanon (0,025 Mol) wurden zusammen mit 4,4 g (0,025 Mol) 1-Benzylpiperazin auf 100°C erhitzt. Anschließend wurden langsam 1,5 ml Ameisensäure unter Rühren zugetropft und weitere 3 h bei 100°C gerührt. Nach dem Abkühlen wurden 10 ml Essigsäureethylester-Hexangemisch zugefügt: nach Stehenlassen über Nacht erhielt man 5,0 g (55 %) reine Verbindung. C₂₄H₂₉N₃, Fp. 120 - 123°C.
d) 4-Piperazinyl-1-phenyl-cyclohexancarbonsäurenitril
   12,7 g (0,035 Mol) des nach a) hergestellten Produkts wurden mit 1 g 10 % Pd auf Kohle-Katalysator und Wasserstoff in salzsaurer methanolischer Lösung bei Atmosphärendruck hydriert. Nach Beendigung der Wasserstoffaufnahme wurde vom Katalysator abfiltriert und die Lösung eingedampft. Nach Behandlung mit Alkali und Extraktion mit Ether wurde die obige Base als cis-trans-Isomerenmischung isoliert. C₁₇H₂₃N₃, Ausbeute 95 %; C₁-H₂₃N₃ x 2 HCl Di-Hydrochlorid, Fp. > 330°C.
e) Cis-4-Piperazinyl-1-phenyl-cyclohexancarbonsäurenitril
   Die Verbindung wurde aus dem nach b) erhaltenen Produkt durch Hydrierung analog d) erhalten. Ausbeute C₁₇H₂₃N₃, Fp. 109 - 113°C.

### B) Herstellung der erfindungsgemäßen Verbindungen

### Verfahren a.

### Beispiel 1

Cis-(4-Cinnamyl)-piperazinyl-1-phenyl-cyclohexancarbonsäurenitril
6,0 g (0,03 Mol) 4-Cyano-4-phenyl-cyclohexanon wurden zusammen mit 6,1 g (0,03 Mol) Cinnamylpiperazin auf 100°C erhitzt. Anschließend wurden langsam 1,5 g (0,033 Mol) Ameisensäure unter Rühren zugetropf und weitere 3 h bei 100°C gerührt. Die Reaktionsmischung wurde sodann mit Essigsäureethylester verdünnt und mit 2N Natronlauge gewaschen. Die Lösungsmittelphase wurde getrocknet und eingedampft. Der Rückstand wurde in Isopropanol aufgenommen und das Hydrochlorid durch Zugabe von isopropanolischer Salzsäure erhalten. Ausbeute: 50 % C₂₆H₃₁N₃ x 2 HCl Di-Hydrochlorid, Fp. > 250°C.

In ähnlicher Weise wurden hergestellt:

### Beispiel 2

cis-4-{4-[3-(4-Fluorphenoxy)-2-hydroxypropyl]piperazinyl}1-(4-fluorphenyl)-cyclohexancarbonsäurenitril C₂₆H₄₁F₂N₃ x 2 HCl Di-Hydrochlorid, Fp. > 250°C.

### Beispiel 3

cis-4-{4-[3-(4-Fluorphenoxy)-2-hydroxypropyl]piperazinyl}1-(2-chlorphenyl)-cyclohexancarbonsäurenitril C₂₆H₃₁ClFN₃O₂ x 2 HCl Di-Hydrochlorid, Fp. > 250°C.

### Beispiel 4

cis-4-{4-[3-Phenoxy-2-hydroxypropyl]piperazinyl}1-(3-methoxyphenyl)-cyclohexancarbonsäurenitril C₂₇H₃₅N₃O₃ x 2 HCl Di-Hydrochlorid, Fp. > 290°C.

### Beispiel 5

cis-4-[4-(3-Phenoxy-2-hydroxypropyl)piperazinyl}1-phenyl-cyclohexancarbonsäurenitril C₂₆H₃₃N₃O₂ x 2 HCl Di-Hydrochlorid, Fp. > 250°C.

### Beispiel 6

cis-4-{4-[3-(4-Fluorphenoxy)-2-hydroxypropyl]-piperazinyl}1-(2-pyridyl)-cyclohexancarbonsäurenitril C₂₅H₃₁FN₄O₂ x 2 HCl Di-Hydrochlorid, Fp. 274 - 278°C.

### Beispiel 7

cis-4-{4-[2-(4-Fluorphenoxy)ethyl]-piperazinyl}1-(2-pyridyl)-cyclohexancarbonsäurenitril C₂₄H₂₉FN₄O x 2 HCl Di-Hydrochlorid, Fp. 282 - 287°C.

### Beispiel 8

cis-4-{4-[3-(4-Fluorphenoxy)-2-hydroxypropyl]-piperazinyl}1-(2-thienyl)-cyclohexancarbonsäurenitril C₂₄H₃₀FN₃O₂S x 2 HCl Di-Hydrochlorid, Fp. > 250°C.

### Beispiel 9

cis-4-{4-[2-(4-Fluorphenoxy)ethyl]-piperazinyl}1-(4-chlorphenyl)-cyclohexancarbonsäurenitril C₂₅H₂₉ClFN₃O₃ x 2 HCl Di-Hydrochlorid, Fp. 270 - 280°C.

### Beispiel 10

cis-4-{4-[3-(4-Fluorphenoxy)-2-hydroxypropyl]-piperazinyl}1-(3-methoxyphenyl)-cyclohexancarbonsäurenitril C₂₇H₃₄FN₃O₃ x 2 HCl Di-Hydrochlorid, Fp. 290 - 300°C.

### Beispiel 11

cis-4-{4-[3-(4-Fluorphenoxy)-2-hydroxypropyl]-piperazinyl}1-(3-chlorphenyl)-cyclohexancarbonsäurenitril C₂₆H₃₁ClFN₃O₂ x 2 HCl Di-Hydrochlorid, Fp. > 250°C.

### Beispiel 12

cis-4-{4-[3-(4-Fluorphenoxy)-2-hydroxypropyl]-piperazinyl}1-(4-methoxyphenyl)-cyclohexancarbonsäurenitril C₂₇H₃₄FN₃O₃ x 2 HCl Di-Hydrochlorid, Fp. > 280°C.

### Beispiel 13

cis-4-{4-[3-(4-Fluorphenoxy)-2-hydroxypropyl]-piperazinyl}1-(2-methoxyphenyl)-cyclohexancarbonsäurenitril C₂₇H₃₄FN₂O₃ x 2 HCl Di-Hydrochlorid, Fp. > 250°C.

### Beispiel 14

cis-4-{4-[3-(4-Fluorphenoxy)-2-hydroxypropyl]-piperazinyl}1-(4-hydroxyphenyl)-cyclohexancarbonsäurenitril C₂₆H₃₂FN₃O₃ x 2 HCl Di-Hydrochlorid, Fp. > 250°C.

### Beispiel 15

4-[4-(3-Phenoxy-propyl]-piperazinyl-1-phenyl-cyclohexancarbonsäurenitril.
5,0 g (0,025 Mol) 4-Cyano-4-phenyl-cyclohexanon und 5,5 g (0,025 Mol) 3-Phenoxypropylpiperazin wurden zusammen mit 3,0 g Essigsäure in 70 ml Ethanol gelöst und anschließend bei Raumtemperatur unter Rühren eine Lösung von 1,7 g (0,027 Mol) Natriumcyanoborhydrid langsam zugetropft. Nach Rühren über Nacht wurde der Ansatz mit Wasser verdünnt und mit Dichlormethan extrahiert. Diese Lösung wurde anschließend mit verdünnter Säure (pH 4), verdünnter Natronlauge und Wasser gewaschen, die organische Phase schließlich getrocknet und eingedampft. Der Rückstand wurde mit isopropanolischer Salzsäure in das Hydrochlorid überführt und aus Isopropanol-Wasser umkristallisiert.
Ausbeute: 50 %, C₂₆H₃₃N₃O x 2 HCl Di-Hydrochlorid, Fp. > 250°C

In ähnlicher Weise wurden die folgenden Verbindungen hergestellt:

### Beispiel 16

4-{4-[3-(4-Fluorphenoxy)-2-hydroxypropyl]-piperazinyl}1-(2-methylphenyl)-cyclohexancarbonsäurentril C₂₇H₃₄FN₃O₂ x 2 HCl Di-Hydrochlorid, Fp. > 250°C.

### Beispiel 17

4-{4-[2-(4-Chlorthiophenoxy)ethyl]-piperazinyl}1-phenylcyclohexancarbonsäurenitril C₂₅H₃₀ClN₃S x 2 HCl Di-Hydrochlorid, Fp. > 270°C.

### Beispiel 18

4-{4-[3-(4-Fluorphenoxy)propyl]-piperazinyl}1-phenyl-cyclohexancarbonsäurenitril C₂₆H₃₂FN₃O x 2 HCl Di-Hydrochlorid, Fp. > 250°C.

Das cis/trans-Isomerengemisch dieser Verbindung wurde präparativ mittels Säulenchromatographie getrennt.
Sorbens: Kieselgel
Elutionsmittel Dichlormethan mit 1,5 % Methanol
Dünnschichtchromatographie: Kieselgel Platten
Laufmittel: Dichlormethan/Methanol 90:10
Laufstrecke: 10 cm
Anfärbemethode: I₂-Dampf

Es wurden erhalten:

### Beispiel 19

cis-4-{4-[3-(4-Fluorphenoxy)propyl]-piperazinyl}1-phenyl-cyclohexancarbonsäurenitril C₂₆H₃₂FN₃O x 2 HCl Di-Hydrochlorid, Fp. > 250°C, Rf = 0,17.

### Beispiel 20

trans-4-{4-[3-(4-Fluorphenoxy)propyl]-piperazinyl}1-phenyl-cyclohexancarbonsäurenitril C₂₆H₃₂FN₃O x 2 HCl Di-Hydrochlorid, Fp. > 250°C, Rf = 0,23 [Methylenchlorid/Methanol] 9:1, Kieselgel.

### Verfahren b.

### Beispiel 21

cis-4-{4-[2-(4-Chlorphenoxy)ethyl]-piperazinyl}1-phenylcyclohexancarbonsäurenitril
5,4 g (0,02 Mol) des Piperazins aus f wurde mit 4,7 g (0,02 Mol) 2-(4-Chlorphenoxy)ethylbromid und 5,5 g Kaliumcarbonat in 100 ml Methylethylketon 4 h unter Rühren zum Sieden erhitzt. Nach dem Abtrennen der Salze wurde das Filtrat eingedampft, der Rückstand in Dichlormethan aufgenommen, mit Wasser gewaschen, getrocknet und im Vakuum eingedampft. Nach Lösen in Isopropanol wurde die Substanz durch Zusatz von isopropanolischer Salzsäure als Hydrochlorid erhalten.
Ausbeute: 60 % C₂₅H₃₀ClN₃O x 2 HCl Di-Hydrochlorid, Fp. > 250°C.

In analoger Weise wurden erhalten:

### Beispiel 22

cis-4-{4-[2-(4-Fluorphenoxy)ethyl]-piperazinyl}1-phenylcyclohexancarbonsäurenitril C₂₅H₃₀FN₃O x 2 HCl Di-Hydrochlorid, Fp. > 260°C,

### Beispiel 23

cis-4-{4-[2-(4-Fluorphenoxy)ethyl]-piperazinyl}1-(4-fluorphenyl)-cyclohexancarbonsäurenitril C₂₅H₂₉F₂N₃O x 2 HCl Di-Hydrochlorid, Fp. > 260°C,

### Beispiel 24

cis-4-{4-[(4-chlorphenoxy) acetyl]-piperazinyl}1-phenylcyclohexancarbonsäurenitril C₂₅H₂₈ClN₃O₂, Fp. 165 - 170°C,

### Beispiel 25

cis-4-{4-[(N-Phenyl-carboxamido)methyl]-piperazinyl}-1-phenyl-cyclohexancarbonsäurenitril C₂₅H₃₀N₄O x 2 HCl Di-Hydrochlorid, Fp. > 300°C,

### Beispiel 26

cis-4-{4-[(N-(2,6-Dimethylphenyl)-carboxamido)methyl]-piperazinyl}-1-phenyl-cyclohexancarbonsäurenitril C₂₇H₃₄N₄O x 2 HCl Di-Hydrochlorid, Fp. 295 - 300°C,

### Beispiel 27

cis-4-{4-[3-Phenoxypropyl]-piperazinyl}-1-phenyl-cyclohexancarbonsäurenitril C₂₆H₃₃N₃O x 2 HCl Di-Hydrochlorid, Fp. > 250°C,

### Verfahren c.

### Beispiel 28

cis-4-{4-[4-Fluorphenoxy]-2-hydroxy-butyl]piperazinyl}-1-phenyl-cyclohexancarbonsäurenitril
3,7 g (0,0147 Mol) des nach Beispiel A.e) hergestellten Piperazins wurden mit 2,5 g (0,0147 Mol) 1,2-Epoxi-4(4-fluorphenoxy)butan in 70 ml Ethanol gelöst und 4 h zum Sieden unter Rückfluß erhitzt. Nach Beendigung der Reaktion wurde der Ansatz mit isopropanolischer Salzsäure versetzt und das erhaltene Hydrochlorid aus einem Isopropanol-Wasser-Gemisch umkristallisiert. Ausbeute: 70 %, C₂₇H₃₄FN₃O₂ x 2 HCl Di-Hydrochlorid, Fp. > 250°C.

In analoger Weise wurden ausgehend vom gemäß A.e) erhaltenen Piperazin durch Umsetzung mit verschiedenen substituierten 1,2-Epoxy-3-phenoxypropanen die folgenden Verbindungen erhalten:

### Beispiel 29

cis-4-{4-[3-Phenoxy-2-hydroxypropyl]piperazinyl}-1-phenyl-cyclohexancarbonsäurenitril C₂₆H₃₃N₃O₂ x 2 HCl Di-Hydrochlorid, Fp. > 250°C,

### Beispiel 30

cis-4-{4-[3-(Phenylmercapto)-2-hydroxypropyl]piperazinyl}-1-phenyl-cyclohexancarbonsäurenitril C₂₆H₃₃N₃OS x 2 HCl Di-Hydrochlorid, Fp. > 250°C,

### Beispiel 31

cis-4-{4-[3-Chlorphenoxy)-2-hydroxy]propyl}piperazinyl}-1-phenyl-cyclohexancarbonsäurenitril C₂₆H₃₂ClN₃O₂ x 2 HCl Di-Hydrochlorid, Fp. > 250°C,

### Beispiel 32

cis-4-{4-[3-(2-Cyanphenoxy)-2-hydroxypropyl]piperazinyl}-1-phenyl-cyclohexancarbonsäurenitril C₂₇H₃₂N₄O₂ x 2 HCl Di-Hydrochlorid, Fp. > 250°C,

### Beispiel 33

cis-4-{4-[3-(4-Nitrophenoxy)-2-hydroxypropyl]piperazinyl}-1-phenyl-cyclohexancarbonsäurenitril C₂₆H₃₂N₄O₄ x 2 HCl Di-Hydrochlorid, Fp. > 250°C,

### Beispiel 34

cis-4-{4-[3-(3-Trifluormethylphenoxy)-2-hydroxypropyl]piperazinyl}-1-phenyl-cyclohexancarbonsäurenitril C₂₇H₃₂F₃N₃O₂ x 2 HCl Di-Hydrochlorid, Fp. > 250°C,

### Beispiel 35

cis-4-{4-[3-(2-Methoxyphenoxy)-2-hydroxypropyl]piperazinyl}-1-phenyl-cyclohexancarbonsäurenitril C₂₇H₃₅N₃O₃ x 2 HCl Di-Hydrochlorid, Fp. > 250°C,

Unter Verwendung von 1,2-Epoxy-3-phenyl-propan wurde erhalten:

### Beispiel 36

cis-4-{4-[3-Phenyl-2-hydroxypropyl]piperazinyl}-1-phenyl-cyclohexancarbonsäurenitril C₂₆H₃₃N₃O x 2 HCl Di-Hydrochlorid, Fp. > 250°C,

In ähnlicher Weise wurde durch Reaktion des Piperazins aus Beispiel e) mit p-Chlorstyroloxid erhalten:

### Beispiel 37

cis-4-{4-[2-(4-Chlorphenyl)-2-hydroxyethyl]piperazinyl}-1-phenyl-cyclohexancarbonsäurenitril C₂₅H₃₀ClN₃O x 2 HCl Di-Hydrochlorid, Fp. > 250°C,

### Beispiel 38

cis-4-{4-[2-(4-Fluorphenyl)-2-hydroxyethyl]piperazinyl}-1-phenyl-cyclohexancarbonsäurenitril C₂₅H₃₀FN₃O x 2 HCl Di-Hydrochlorid, Fp. > 300°C,

### Beispiel 39

cis-4-{4-[3,3-Dimethyl-3-phenyl-2-hydroxypropyl]piperazinyl}-1-phenyl-cyclohexancarbonsäurenitril C₂₈H₃₇N₃O x 2 HCl Di-Hydrochlorid, Fp. > 300°C,

### Beispiel 40

cis-4-{4-[3(4-Chlorphenoxy)-2-t-butyl-2-hydroxypropyl]piperazinyl}-1-phenyl-cyclohexancarbonsäurenitril C₃₀H_{4O}ClN₃O₂ x 2 HCl Di-Hydrochlorid, Fp. 285 - 290°C,

### Beispiel 41

cis-4-{4-[3-Phenoxy-2-hydroxy-2-methyl]piperazinyl}-1-phenyl-cyclohexancarbonsäurenitril C₂₇H₃₅N₃O₂ x 2 HCl Di-Hydrochlorid, Fp. > 270°C,

### Beispiel 42

cis-4-{4-[3-(2,4-Dichlorphenoxy-2-hydroxy-2-methyl-propyl]piperazinyl}-1-phenyl-cyclohexancarbonsäurenitril C₂₇H₃₃Cl₂N₃O₂ x 2 HCl Di-Hydrochlorid, Fp. > 270°C,

### Beispiel 43

cis-4-{4-[3-(4-Acetyl-phenoxy)-2-hydroxypropyl]piperazinyl}-1-phenyl-cyclohexancarbonsäurenitril C₂₈H₃₅N₃O₃ x 2 HCl Di-Hydrochlorid, Fp. > 280°C,

### Beispiel 44

cis-4-{4-[3-(2-Carbomethoxy)-phenoxy-2-hydroxypropyl]piperazinyl}-1-phenyl-cyclohexancarbonsäurenitril C₂₈H₃₅N₃O₄ x 2 HCl Di-Hydrochlorid, Fp. 250 - 260°C,

Durch Umsetzung des gemäß A.d) erhaltenen cis/trans-Gemisches des Piperazins mit (+)-1,2-Epoxy-3-(4-fluorphenoxy)propan analog Beispiel 28 wurde ein cis/trans-Gemisch erhalten, das mittels präparativer Säulenchromatographie wie in Beispiel 18 beschrieben, getrennt wurde.

Es wurde isoliert:

### Beispiel 45

(-)trans-4-{4-[3-(4-Fluorphenoxy)-(S)-2-hydroxypropyl]piperazinyl}-1-phenyl-cyclohexancarbonsäurenitril C₂₆H₃₂FN₃O₃ x 2 HCl Di-Hydrochlorid, Fp. > 250°C, [a]²⁰₅₈₉ₙₘ = -12,1° (C = 10 mg/ml, Methanol 95 %) Rf = 0,4

### Beispiel 46

(-)cis-4-{4-[3-(4-Fluorphenoxy)-(S)-2-hydroxypropyl]piperazinyl}-1-phenyl-cyclohexancarbonsäurenitril
a) C₂₆H₃₂FN₃O₂ x 2 HCl Di-Hydrochlorid, Fp. > 250°C,
b) C₂₆H₃₂FN₃O₂ x 2 CH₃SO₃H Bis-Methansulfonat Fp. > 250°C,
C) C₂₆H₃₂FN₃O₂ Base, Fp. > 250°C, [a]²⁰₅₈₉ₙₘ = -10,1° (C = 10 mg/ml, Methanol 95 %) Rf = 0,3

Durch analoge Umsetzung mit (-)-1,2-Epoxy-3-(4-fluorphenoxy)propan und nachfolgende chromatographische Trennung wurden erhalten:

### Beispiel 47

(+)-trans-4-{4-[3-(4-Fluorphenoxy)-(R)-2-hydroxypropyl]-piperaziny}-1-phenyl-cyclohexancarbonsäurenitril C₂₆H₃₂FN₃O₂ x 2 HCl Di-Hydrochlorid, Fp. > 250°C [a]²⁰₅₈₉ₙₘ = 11,1° (C = 10 mg/ml, Methanol 95 %) Rf = 0,4

### Beispiel 48

(+)-cis-4-{4-[3-(4-Fluorphenoxy)-(R)-2-hydroxypropyl]-piperaziny}-1-phenyl-cyclohexancarbonsäurenitril C₂₆H₃₂FN₃O₂ x 2 HCl Di-Hydrochlorid, Fp. > 250°C
C₂₆H₃₂FN₃O₂ Base, Fp. 143-149°C
[a]²⁰₅₈₉ₙₘ = 9,6° (C = 10 mg/ml, Methanol 95 %)

Wurde die Umsetzung mit reinem cis-Piperazin des Beispiels A.e) und (+)- oder (-)-1,2-Epoxy-3-(4-fluorphenoxy)propan durchgeführt, so wurden die Substanzen der Beispiele 46 und 48 in einer Ausbeute von 70 % erhalten.

### Beispiel 49

cis(-)-4-{4-[3-(4-Fluorphenoxy)-2-acetoxypropyl]-piperaziny}-1-phenyl-cyclohexancarbonsäurenitril
3 g der Base aus Beispiel 29 wurden mit 30 ml Essigsäureanhydrid 1,5 h unter Rückfluß erhitzt, anschließend das überschüssige Essigsäureanhydrid im Vakuum abdestilliert. Der Rückstand wurde in Diisopropylether gelöst und die Substanz mit etherischer Salzsäure als Di-Hydrochlorid gewonnen.
C₂₈H₃₄FN₃O₂ x2 HCl Di-Hydrochlorid, Fp. 272-275°C

### Beispiel 50

cis-(-)-4-{4-[3-(4-Fluorphenoxy)-2-methoxypropyl]piperazinyl}-1-phenyl-cyclohexancarbonsäurenitril
4,4 g (0,01 Mol) der Base aus Beispiel 29 wurden mit 0,3 g 80-prozentigem Natriumhydrid in 50 ml DMF und 1,4 g (0,01 Mol) Methyljodid bei 25-40°C 12 h gerührt. Nach dem Ende der Reaktion wurde mit 250 ml Wasser verdünnt, die Base mit Methylenchlorid extrahiert und nach dem Trocknen der Lösung und Abdestillieren des Lösungsmittels der Rückstand mit isopropanolischer Salzsäure in das Di-Hydrochlorid überführt:
C₂₇H₃₄FN₃O₂ x 2 HCl Di-Hydrochlorid, Fp. 270-280°C

## Patentansprüche

1. 1-Aryl-4-piperazinyl-cyclohexancarbonsäurenitrile der Formel I 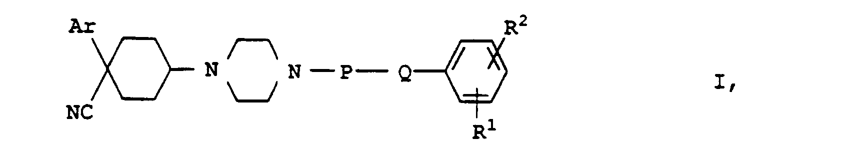 worin
Ar einen gegebenenfalls durch ein Chloratom, eine Methyl-, Methoxy- oder Trifluormethylgruppe, oder durch ein oder zwei Fluoratome substituierten Phenylrest, eine 2- oder 3-Thienylgruppe oder eine 2-, 3- oder 4-Pyridylgruppe,
R¹ ein Wasserstoff-, Fluor- oder Chloratom oder eine Trifluormethyl-, Methoxy-, Methyl-, Nitro-, Cyano-, Acetyl-, Methoxycarbonyl- oder Ethoxycarbonylgruppe,
R² ein Wasserstoff- oder Chloratom oder eine Methylgruppe,
P ein geradkettiger oder verzweigter Kohlenwasserstoffrest mit 2 bis 8 C-Atomen, der durch eine Oxo-, Hydroxy, Methoxy- oder Acetoxygruppe substituiert sein kann, und
Q ein Sauerstoff- oder Schwefelatom, eine NH-Gruppe oder eine direkte Bindung
darstellen, sowie deren Salze mit physiologisch verträglichen Säuren.

2. (-)-cis-4-{4-[3-(4-Fluorphenoxy)-(S)-2-hydroxypropyl]piperazinyl}-1-phenyl-cyclohexancarbonsäurenitril nach Anspruch 1.

3. (-)-trans-4-{4-[3-(4-Fluorphenoxy)-(S)-2-hydroxypropyl]piperazinyl}-1-phenyl-cyclohexancarbonsäurenitril nach Anspruch 1.

4. 1-Aryl-4-piperazinyl-cyclohexancarbonsäurenitrile der Formel I gemäß Anspruch 1 zur Verwendung bei der Bekämpfung von Krankheiten.

5. Verfahren zur Herstellung der 1-Aryl-4-piperazinylcyclohexancarbonsäurenitrile der Formel I 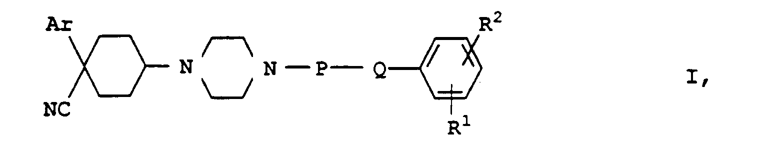 worin
Ar einen gegebenenfalls durch ein Chloratom, eine Methyl-, Hydroxy-, Methoxy- oder Trifluormethylgruppe, oder durch ein oder zwei Fluoratome substituierten Phenylrest, eine 2- oder 3-Thienylgruppe oder eine 2-, 3- oder 4-Pyridylgruppe,
R¹ ein Wasserstoff-, Fluor- oder Chloratom oder eine Trifluormethyl-, Methoxy-, Methyl-, Nitro-, Cyano-, Acetyl-, Methoxycarbonyl- oder Ethoxycarbonylgruppe,
R² ein Wasserstoff- oder Chloratom oder eine Methylgruppe,
P ein geradkettiger oder verzweigter Kohlenwasserstoffrest mit 2 bis 8 C-Atomen, der durch eine Hydroxy, Methoxy- oder Acetoxygruppe substituiert sein kann, und
Q ein Sauerstoff- oder Schwefelatom, eine NH-Gruppe oder eine direkte Bindung
darstellen, sowie deren Salze mit physiologisch verträglichen Säuren, dadurch gekennzeichnet, daß man entweder
a) eine Verbindung der Formel II 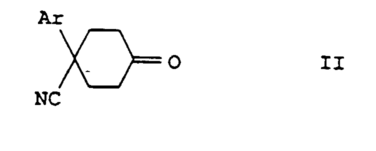 in welcher Ar, die angegebene Bedeutung hat, mit einem Piperazinderivat III 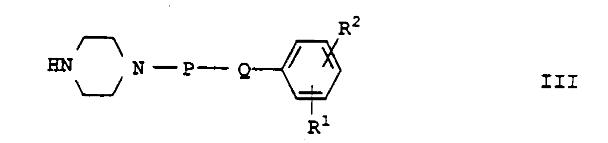 in welchem P, Q, R¹ und R² die angegebene Bedeutung haben, oder
b) eine Verbindung der Formel IV 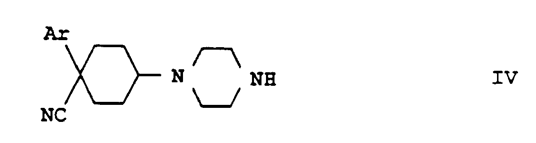 in welcher Ar die oben genannte Bedeutung hat, mit einer Verbindung der Formel V 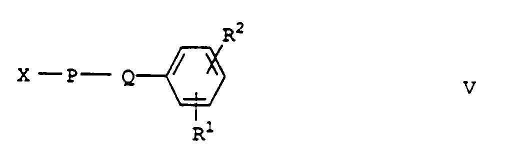 umsetzt, wobei P, R1 und R2 die obengenannte Bedeutung haben und X einen reaktionsfähigen Säurerest darstellt, oder
c) - falls P durch eine Hydroxygruppe substituiert ist und Q keine NE-Gruppe darstellt - eine Verbindung der Formel IV mit einer Verbindung der Formel VI 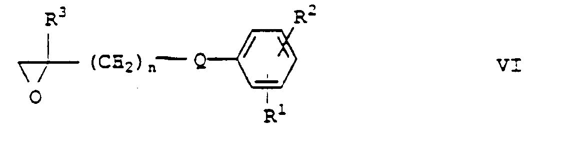 umsetzt, wobei Q, R¹ und R² die vorstehend genannte Bedeutung haben, R³ einen C₁₋₄-Alkylrest darstellt und n die Zahl 1 oder 2 bedeutet,
und die so erhaltenen Verbindungen gegebenenfalls anschließend mit physiologisch verträglichen Säuren in ihre Salze überführt.

## Claims

1. A 1-aryl-4-piperazinylcyclohexanecarbonitrile of the formula I 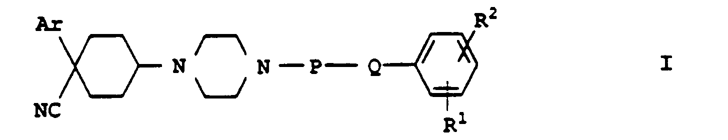 where
Ar is phenyl which is unsubstituted or monosubstituted by chlorine, methyl, methoxy or trifluoromethyl or mono- or disubstituted by fluorine, or is 2- or 3-thienyl or 2-, 3- or 4-pyridyl,
R¹ is hydrogen, fluorine, chlorine, trifluoromethyl, methoxy, methyl, nitro, cyano, acetyl, methoxycarbonyl or ethoxycarbonyl,
R² is hydrogen, chlorine or methyl,
P is straight-chain or branched alkylene of from 2 to 8 carbons, which can be monosubstituted by oxo, hydroxyl, methoxy or acetoxy, and
Q is oxygen or sulfur, NH or a bond,
or the salts thereof with physiologically tolerated acids.

2. (-)-cis-4-{4-[3-(4-Fluorophenoxy)-(S)-2-hydroxypropyl]piperazinyl}-1-phenylcyclohexanecarbonitrile as claimed in claim 1.

3. (-)-trans-4-{4-[3-(4-Fluorophenoxy)-(S)-2-hydroxypropyl]piperazinyl}-1-phenylcyclohexanecarbonitrile as claimed in claim 1.

4. A 1-aryl-4-piperazinylcyclohexanecarbonitrile of the formula I as claimed in claim 1 for use in controlling diseases.

5. A process for preparing a 1-aryl-4-piperazinylcyclohexanecarbonitrile of the formula I 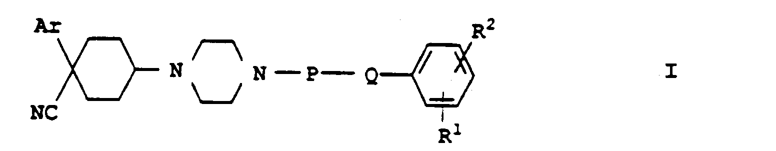 where
Ar is phenyl which is unsubstituted or monosubstituted by chlorine, methyl, hydroxyl, methoxy or trifluoromethyl or mono- or disubstituted by fluorine, or is 2- or 3-thienyl or 2-, 3- or 4-pyridyl,
R¹ is hydrogen, fluorine, chlorine, trifluoromethyl, methoxy, methyl, nitro, cyano, acetyl, methoxycarbonyl or ethoxycarbonyl,
R² is hydrogen, chlorine or methyl,
P is straight-chain or branched alkylene of from 2 to 8 carbons, which can be monosubstituted by hydroxyl, methoxy or acetoxy, and
Q is oxygen or sulfur, NH or a bond,
and the salts thereof with physiologically tolerated acids, which comprises either
a) reacting a compound of the formula II 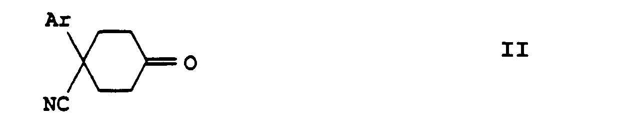 where Ar has the stated meanings, with a piperazine derivative III 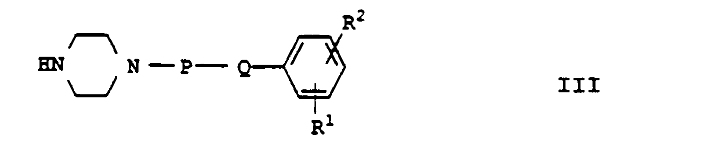 where P, Q, R¹ and R² have the stated meanings, or
b) reacting a compound of the formula IV 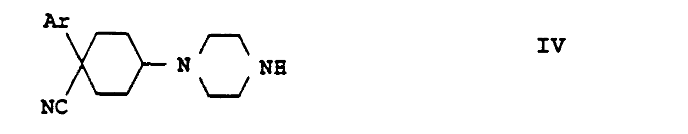 where Ar has the abovementioned meanings, with a compound of the formula V 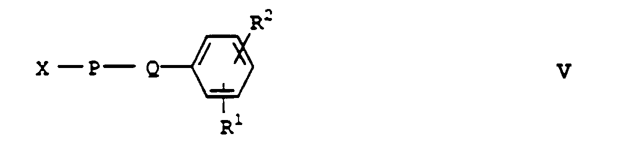 where P, R¹ and R² have the abovementioned meanings, and X is a reactive acid residue, or
c) if P is monosubstituted by hydroxyl, and Q is not NH, reacting a compound of the formula IV with a compound of the formula VI 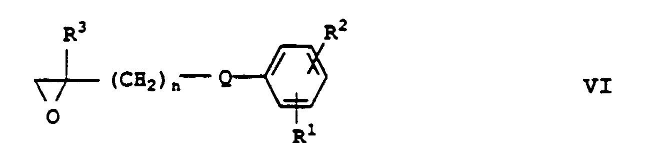 where Q, R¹ and R² have the abovementioned meanings, R³ is C₁₋₄-alkyl, and n is 1 or 2,
and subsequently converting the resulting compounds where appropriate with physiologically tolerated acids into their salts.

## Revendications

1. Nitriles d'acides 1-aryl-4-pipérazinyl-cyclohexanecarboxyliques de formule I 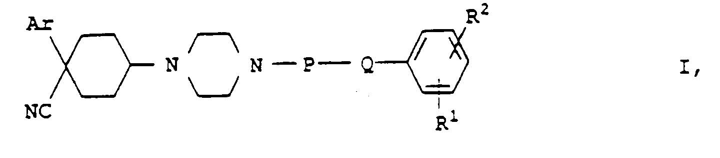 dans laquelle
Ar représente un reste phényle éventuellement substitué par un atome de chlore, un groupe méthyle, méthoxy ou trifluorométhyle, ou par un ou deux atomes fluor, ou représente un groupe 2- ou 3-thiényle ou un groupe 2-, 3- ou 4-pyridyle,
R¹ représente un atome d'hydrogène, de fluor ou de chlore ou un groupe trifluorométhyle, méthoxy, méthyle, nitro, cyano, acétyle, méthoxycarbonyle ou éthoxycarbonyle,
R² représente un atome d'hydrogène ou de chlore ou un groupe méthyle,
P représente un reste d'hydrocarbure linéaire ou ramifié ayant 2 à 8 atomes C, qui peut être substitué par un groupe oxo, hydroxy, méthoxy ou acétoxy, et
Q est un atome d'oxygène ou de soufre, un groupe NH ou une liaison directe,
ainsi que leurs sels avec des acides physiologiquement acceptables.

2. Nitrile d'acide (-)-cis-4-{4-[3-(4-fluorophénoxy)-(S)-2-hydroxypropyl]pipérazinyl}-1-phényl-cyclohexanecarboxylique selon la revendication 1.

3. Nitrile d'acide (-)-trans-4-{4-[3-(4-fluorophénoxy)-(S)-2-hydroxypropyl]pipérazinyl}-1-phényl-cyclohexanecarboxylique selon la revendication 1.

4. Nitriles d'acides 1-aryl-4-pipérazinyl-cyclohexanecarboxyliques de formule I, selon la revendication 1, pour utilisation à la lutte contre des maladies.

5. Procédé de préparation des nitriles d'acides 1-aryl-4-pipérazinylcyclohexanecarboxyliques de formule I 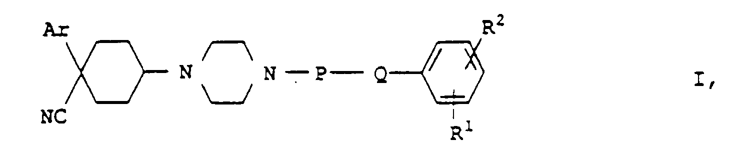 dans laquelle
Ar représente un reste phényle éventuellement substitué par un atome de chlore, un groupe méthyle, hydroxy, méthoxy ou trifluorométhyle, ou par un ou deux atomes fluor, ou représente un groupe 2- ou 3-thiényle ou un groupe 2-, 3- ou 4-pyridyle,
R¹ représente un atome d'hydrogène, de fluor ou de chlore ou un groupe trifluorométhyle, méthoxy, méthyle, nitro, cyano, acétyle, méthoxycarbonyle ou éthoxycarbonyle,
R² représente un atome d'hydrogène ou de chlore ou un groupe méthyle,
P représente un reste d'hydrocarbure linéaire ou ramifié ayant 2 à 8 atomes C, qui peut être substitué par un groupe hydroxy, méthoxy ou acétoxy, et
Q est un atome d'oxygène ou de soufre, un groupe NH ou une liaison directe,
ainsi que leurs sels avec des acides physiologiquement acceptables, caractérisé par le fait que l'on met à réagir, soit
a) un composé de formule II 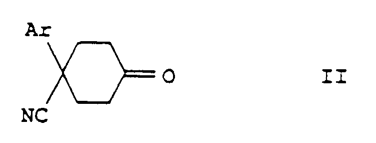 dans laquelle Ar a la signification donnée plus haut, avec un dérivé de pipérazine III 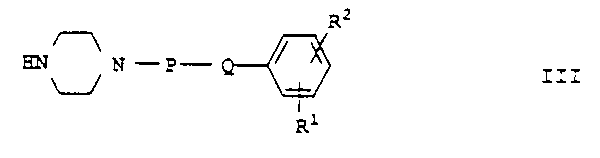 dans laquelle P, Q, R¹ et R² ont la signification donnée plus haut, soit
b) un composé de formule IV 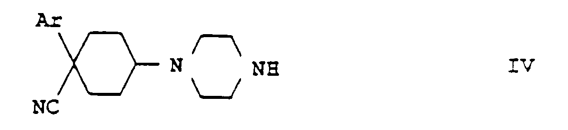 dans laquelle Ar a la signification donnée plus haut, avec un composé de formule V 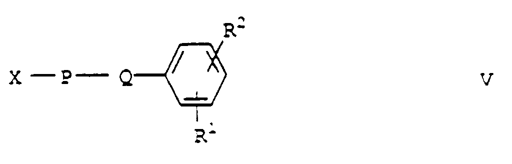 P, R¹ et R² ayant la signification donnée plus haut et X représentant un reste d'acide réactif,
ou c) - au cas où P est substitué par un groupe hydroxy et Q ne représente pas de groupe NH - on met à réagir un composé de formule IV avec un composé de formule VI 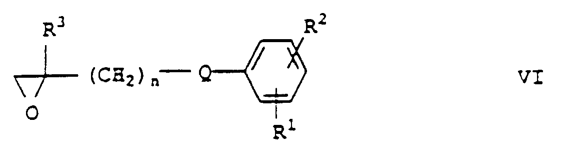 Q, R¹ et R² ayant la signification donnée plus haut, R³ représentant un reste alkyle en C1-C4 et n représentant un des nombres 1 ou 2, et, le cas échéant, on transforme ensuite les composés ainsi obtenus en leurs sels avec des acides physiologiquement acceptables.
